# EUROPÄISCHE PATENTSCHRIFT

(11) **EP 2 729 444 B1**
(45) Veröffentlichungstag und Bekanntmachung des Hinweises auf die Patenterteilung: **23.08.2017**
(21) Anmeldenummer: 12734907.4
(22) Anmeldetag: 05.07.2012
(51) Int. Cl.: C07C 255/58, C07C 227/16, C07C 253/14, C07C 253/30

(54) **VERFAHREN ZUR HERSTELLUNG VON 2-AMINO-5-CYANO-N,3-DIMETHYLBENZAMID**
METHOD FOR MANUFACTURING 2-AMINO-5-CYANO-N,3-DIMETHYLBENZAMIDE
PROCÉDÉ DE FABRICATION DE LA 2-AMINO-5-CYANO-N, 3-DIMÉTHYLBENZAMIDE

(30) Priorität: 08.07.2011 EP 11173323; 11.07.2011 US 201161506263 P
(43) Veröffentlichungstag der Anmeldung: 14.05.2014
(73) Patentinhaber: Bayer Intellectual Property GmbH, 40789 Monheim (DE)
(72) Erfinder: VOLZ, Frank, 50825 Köln (DE); HIMMLER, Thomas, 51519 Odenthal (DE); MÜLLER, Thomas Norbert, 40789 Monheim (DE); LEHMANN, Sandra, 51373 Leverkusen (DE); VON MORGENSTERN, Sascha, 51399 Burscheid (DE); MORADI, Wahed Ahmed, 40789 Monheim (DE); PAZENOK, Sergii, 42699 Solingen (DE); LUI, Norbert, 51519 Odenthal (DE)
(74) Vertreter: BIP Patents
(86) Internationale Anmeldenummer: PCT/EP2012/063167
(87) Internationale Veröffentlichungsnummer: WO 2013/007603

(56) Entgegenhaltungen:
- WO-A1-2009/111553
- US-A1- 2004 229 900

## Beschreibung

Die vorliegende Erfindung betrifft ein Verfahren zur Herstellung von 2-Amino-5-cyano-N,3-dimethylbenzamid der Formel (I) 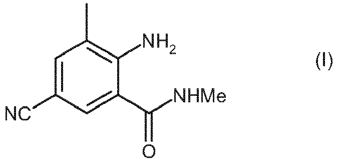 durch Umsetzung von 2-Amino-5-cyano-3-methylbenzoesäureestern bzw. -diestern mit Methylamin, wobei die 2-Amino-5-cyano-3-methylbenzoesäureester bzw. -diester aus den entsprechenden BromVerbindungen durch Cyanierung mit Kupfer(I)-cyanid erhalten werden können. Die Bromverbindungen lassen sich durch Bromierung mit einer Mischung aus Bromwasserstoff / Wasserstoffperoxid einfach herstellen.

In der Literatur sind bereits Herstellungsverfahren beschrieben, welche auch die Verbindung der Formel (I) umfassen (vgl. z.B. WO 2008/08252, WO 2009/085816, WO 2009/006061, WO 2009/061991, WO 2009/111553, WO 2008/070158, WO 2008/082502) und zu unterschiedlicher Reinheit und Ausbeute führen.

Die Aufgabe der vorliegenden Erfindung ist daher die Bereitstellung neuer, wirtschaftlicher Verfahren zur Herstellung von 2-Amino-5-cyano-N,3-dimethylbenzamid der Formel (I) in höherer Reinheit, Ausbeute und besserer Qualität, wobei insbesondere die Aufreinigung der hierfür benötigten 2-Amino-5-cyano-3-methylbenzoesäureester bzw. -diester im Vordergrund steht.

Die Aufgabe wurde gemäß der vorliegenden Erfindung gelöst durch ein Verfahren gemäß Anspruch 1 zur Herstellung von 2-Amino-5-cyano-N,3-dimethylbenzamid der allgemeinen Formel (I) 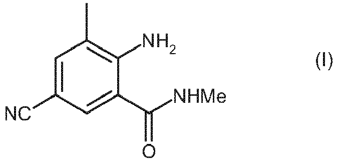 durch Umsetzung von 2-Amino-5-cyano-3-methylbenzoesäureestern der Formel (II) 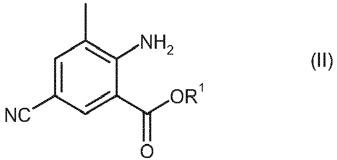 in welcher
- R¹: für Alkyl, Cycloalkyl Alkoxyalkyl, Arylalkyl, Thioalkyl, Alkylthioalkyl, Alkylsulfonylalkyl, Cyanoalkyl, Haloalkyl, Nitroalkyl oder Aryl steht,
- R¹: bevorzugt für Methyl, Ethyl, (C₅-C₁₂)Alkyl oder Aryl steht,
- R¹: besonders bevorzugt für Methyl, Ethyl, Pentyl, Hexyl, oder 2-Ethylhexyl steht,
- R¹: ganz besonders bevorzugt für Pentyl, Hexyl, oder 2-Ethylhexyl steht,
oder durch Umsetzung von 2-Amino-5-cyano-3-methylbenzoesäurediestern der Formel (III) 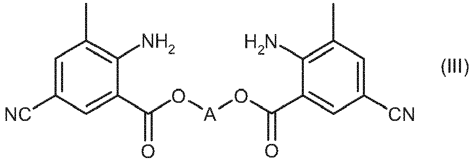 in welcher
- A: für Alkylen und Alkoxyalkylen steht,
- A: bevorzugt für Methylen oder Ethylen, Hexylen steht,
- A: besonders bevorzugt für Ethylen oder Hexylen steht,
mit MeNH₂ ohne weitere Zusätze oder unter Verwendung von mindestens einer Base wie Natriumamid, Natriumhydrid, Natriumhydroxid, Natriumcyanid, Kaliumcyanid, Kaliumcarbonat, Natriumcarbonat, Cäsiumcarbonat, Natriummethylat oder Triethylamin.

Die 2-Amino-5-cyano-3-methylbenzoesäureester bzw. -diester der Formeln (II) und (III) können durch Umsetzung von Verbindungen der Formel (IV) oder der Formel (V) 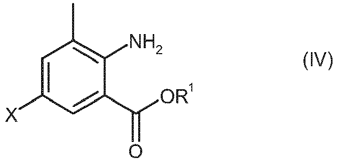 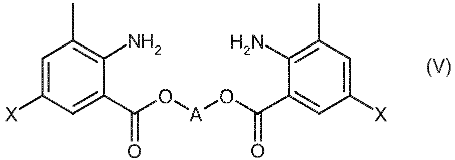 in welcher jeweils
- X: für Br oder I steht und A die oben angegebenen Bedeutung hat,
mit Kupfer(I)-cyanid erhalten werden.

Die halogenierten Ester bzw. Diester der allgemeinen Formeln (IV) und (V) können durch Umsetzung der Verbindungen der allgemeinen Formeln (VI) und (VII) 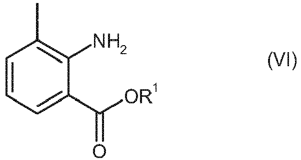 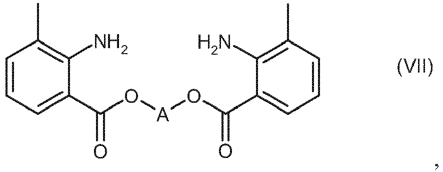 wobei R¹ und A die oben angegebenen Bedeutungen haben,
mit einer Mischung aus Bromwasserstoff und Wasserstoffperoxid erhalten werden.

Das erfindungsgemäße Verfahren kann anhand des folgenden Schemas (I) erläutert werden 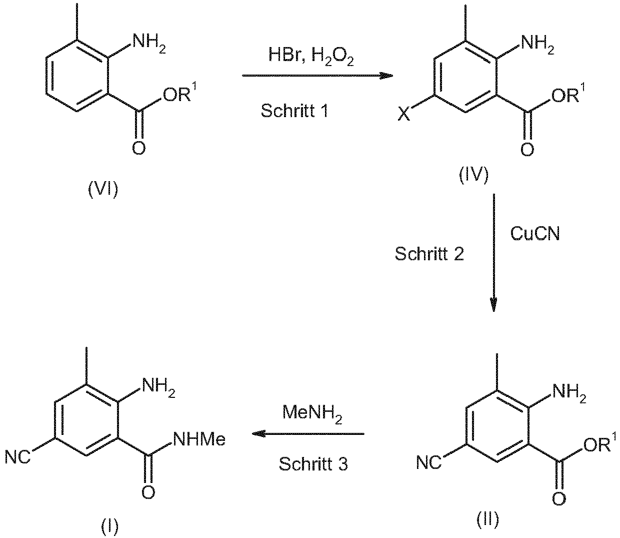 bzw. 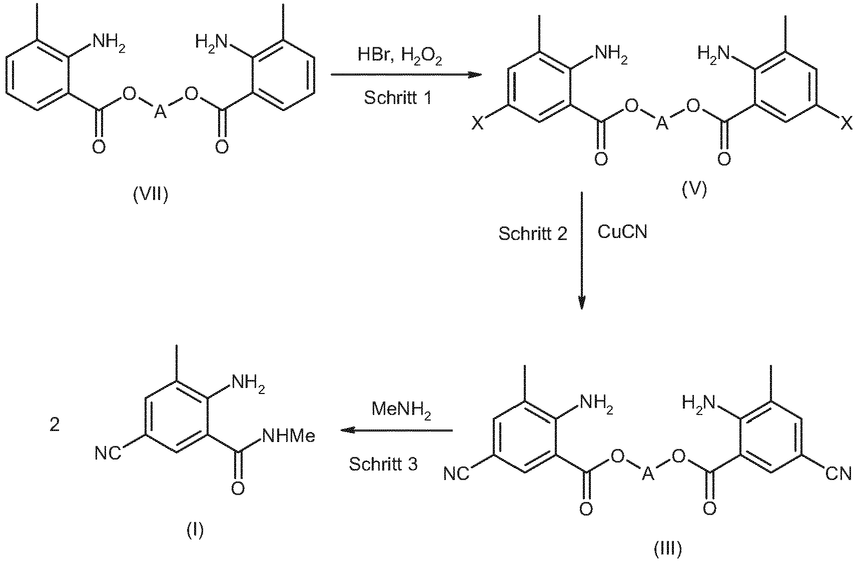 in welcher R¹, X und A die oben angegebenen allgemeinen Bedeutungen haben.

### Allgemeine Definitionen:

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Jod, wobei Fluor, Chlor und Brom bevorzugt und Chlor und Brom besonders bevorzugt verwendet werden. Substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen = (Halogenalkyl-Gruppen) sind beispielsweise ausgewählt aus Trifluormethyl (CF₃), Difluormethyl (CHF₂), CCl₃, CFCl₂, CF₃CH₂, ClCH₂, CF₃CCl₂.

Alkyl-Gruppen sind im Zusammenhang mit der vorliegenden Erfindung, soweit nicht abweichend definiert, lineare oder verzweigte Kohlenwasserstoff-Gruppen. Alkylgruppen können im Zusammenhang mit der vorliegenden Erfindung ein- oder mehrfach durch weitere Gruppen substituiert sein, beispielsweise sind Cyanoalkyl-Gruppen ausgewählt aus Cyano-methyl, Cyano-ethyl, etc., Nitro-alkylgruppen sind beispielsweise ausgewählt aus Nitro-methyl, Nitro-ethyl, etc.. Alkoxyalkylgruppen sind durch Alkoxy substitutierte Alkylgruppen, im einzelnen sind beispielsweise die Bedeutungen Methoxymethyl, Ethoxymethyl, Propoxyethyl, etc. umfasst.

Die Definition Alkyl und C₁-C₁₂-Alkyl umfasst beispielsweise die Bedeutungen Methyl, Ethyl, n-, isoPropyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Aryl-Reste sind im Zusammenhang mit der vorliegenden Erfindung, soweit nicht abweichend definiert, aromatische Kohlenwasserstoff-Reste, die ein, zwei oder mehrere Heteroatome, die ausgewählt sind aus O, N, P und S aufweisen können und optional durch weitere Gruppen substituiert sein können.

Cycloalkyl-Gruppen sind im Zusammenhang mit der vorliegenden Erfindung, soweit nicht abweichend definiert, ringförmige gesättigte Kohlenwasserstoff-Gruppen.

Arylalkyl-Gruppen und Arylalkoxy-Gruppen sind im Zusammenhang mit der vorliegenden Erfindung, soweit nicht abweichend definiert, durch Aryl-Gruppen substituierte Alkyl- bzw. Alkoxy-Gruppen, die eine Alkylenkette aufweisen können. Im Einzelnen umfasst die Definition Arylalkyl beispielsweise die Bedeutungen Benzyl- und Phenylethyl-; die Definition Arylalkoxy bespielsweise die Bedeutung Benzyloxy.

Alkylen-Gruppen sind im Zusammenhang mit der vorliegenden Erfindung, soweit nicht abweichend definiert, lineare oder verzweigte C₁-C₁₀-Akylenketten.

### Schritt 1

Bromanthranilsäureester (X = Br) der Formeln (IV) und (V) können folgendermaßen hergestellt werden:

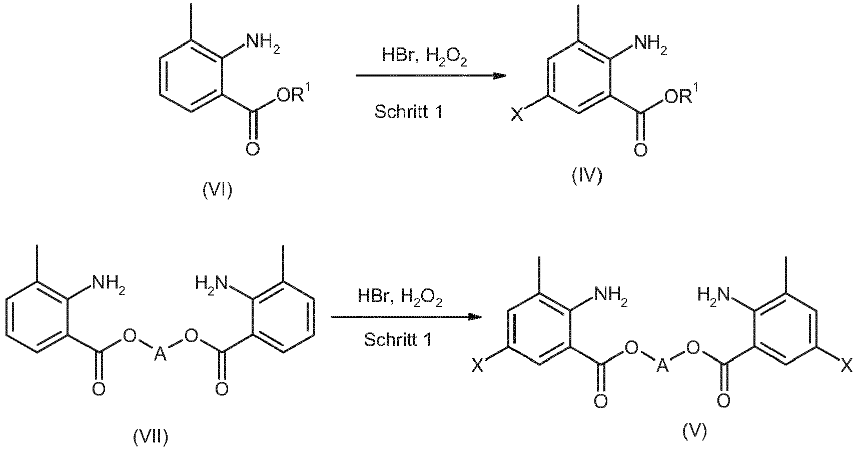

Die Herstellung von in 5er Position bromierten Anthranilsäurederivaten erfolgt meist mit elementarem Brom in flüssiger oder gasförmiger Form (Helv. Chim. Acta 2004, 87, 1333-1356; WO 2008/065508; WO 2006/062978; WO 2008/070158; WO 2010/149359). Da die erhaltenen Anthranilsäureester aufgrund der HBr-Bildung als entsprechende Ammoniumbromide anfallen und für deren Freisetzung einen zusätzlichen Aufreinigungsschritt erfordern, erscheint es von Vorteil die Kombination von Bromwasserstoff und Wasserstoffperoxid zu verwenden. Die bei dieser Variante umgesetzten Ester konnten überraschenderweise ohne störende Hydrolyse und zu erwartende Esterspaltung in sehr hoher Reinheit und in hoher chemischer Ausbeute erhalten werden. Des Weiteren erfordert diese Umsetzung als Lösungsmittel lediglich Wasser und keinerlei Zusätze wie Essigsäure und dergleichen wie in der Literatur beschrieben.

Die Durchführung des erfindungsgemäßen Verfahrensschritts erfolgt vorzugsweise innerhalb eines Temperaturbereichs von 40°C bis +120°C, besonders bevorzugt bei Temperaturen von 40°C bis + 80 °C.

Der erfindungsgemäße Verfahrensschritt (1) wird im Allgemeinen unter Normaldruck durchgeführt. Alternativ ist es jedoch auch möglich, im Vakuum zu arbeiten oder unter Druck.

Die Reaktionszeit ist nicht kritisch und kann, in Abhängigkeit von der Ansatzgröße und von der Temperatur, in einem Bereich zwischen unter einer und mehreren Stunden gewählt werden.

Bei der Durchführung des erfindungsgemäßen Verfahrensschritts setzt man für 1 Mol des Esters der Formel (VI) bzw. (VII) 0,8 Mol bis 1,4 Mol, vorzugsweise 0,9 Mol bis 1,2 Mol, besonders bevorzugt 1,05 Mol Bromwasserstoff und 0,8 Mol bis 1,4 Mol, vorzugsweise 0,9 Mol bis 1,2 Mol, besonders bevorzugt 1,1 Mol Wasserstoffperoxid ein.

Geeignete Lösungsmittel sind beispielsweise aliphatische, alicyclische oder aromatische halogenierte Kohlenwasserstoffe, wie z.B. Chlorbenzol, Dichlorbenzol, Dichlormethan, Chloroform, Tetrachlormethan, Dichlorethan oder Trichlorethan, Nitrile, wie Acetonitril, Propionitril, n- oder iso-Butyronitril oder Benzonitril und Wasser, aliphatische oder alicyclische Carbonsäuren. Besonders bevorzugt verwendet man Chlorbenzol, Dichlorbenzol, Dichlormethan, Chloroform, Dichlorethan, Trichlorethan, Essigsäure, Propionsäure, Butansäure, Acetonitril, Butyronitril und Wasser.

Die bromierten Produkte können ohne vorherige Aufarbeitung entweder aus der wässrigen Phase durch Filtration oder je nach Schmelzpunkt des erhaltenen Produktes in Form der Schmelze aus dem 2-Phasen System abgetrennt werden. Die erhaltenen Produkte können ohne weitere Aufreinigung im darauffolgenden Schritt (2), in welchem die Cyanierung stattfindet, eingesetzt werden.

### Schritt 2

Cyanoanthranilsäureester der Formeln (IV) und (V) können folgendermaßen hergestellt werden:

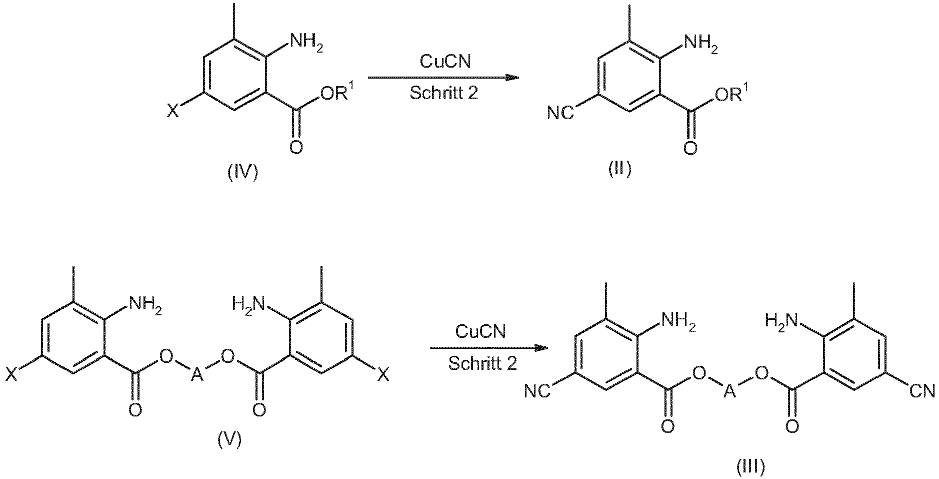

In der Literatur wurde bereits beschrieben, dass Cyanoanthranilsäureester durch Umsetzung von in 5er Position halogenierten Anthranilsäurederivaten mit katalytischen Mengen an Palladium-, Nickel- und Kupferkatalysatoren unter Zusatz von verschiedenen Diamin-, Pyridin- und Phosphinliganden (WO 2008/070158 A1, WO 2008/082502 A2, WO 2009/006061 A2, WO 2009/061991 A1, WO 2009/085816 A1, WO 2009/111553 A1) erhältlich sind. Diese Verfahren bringen allerdings folgende Nachteile mit sich: Die homogenen Katalysatoren auf Palladium-, Nickel- und Kupferbasis sind meist schwer von dem zu isolierenden Produkt abtrennbar und deshalb auch nur schwer zu rezyklisieren. Gleiches gilt für die eingesetzten Liganden. Darüber hinaus sind Nickelkatalysatoren aufgrund ihrer Toxizität bedenklich, sollten sie als Verunreinigung ins Produkt verschleppt werden. Daraus resultiert oft ein aufwendiges Aufreinigungsverfahren, das zu hohen Kosten führt. Bei der Palladium- und Nickelkatalyse erfolgt zudem meist eine Dehalogenierung und man findet anstatt des eingebauten Cyanids nur ein Proton am Aromaten. Darüber hinaus sind in der Literatur keine Beispiele für Cyanierungen von 2-Amino-5-brom-3-methylbenzoesäurealkylester mit stöchiometrischen Mengen an Kupfer(I)-cyanid beschrieben. Einziges experimentell belegtes Beispiel für die Cyanierung von 2-Amino-5-brom-3-ethylbenzoesäureethylester: V. A. Sniekus et al. J. Org. Chem. 1972, 37, 2845-2848. Die Aufreinigung erfordert oft aufwendige Reinigungsschritte (G. P. Ellis et al., Chem. Rev. 1987, 87, 779-794; Friedman, Schechter, J. Org. Chem. 1961, 26, 2522-2524). Die in Formel (II) dargestellten Substitutionsmuster für R = C₁-C₄Alkyl sind in den folgenden Literaturstellen vorbeschrieben: WO 2008/070158 A1, WO 2008/082502 A2, WO 2009/006061 A2, WO 2009/061991 A1, WO 2009/085816 A1, WO 2009/111553 A1. In diesen Offenlegungsschriften werden allerdings keine experimentell belegte Beispiele für einen Brom-Cyan-Austausch an einem Anthranilsäureester der Formel (IV) bzw. (V) beschrieben.. Überraschenderweise konnte gefunden werden, dass im Gegensatz zu den oben beschriebenen Verfahren bei Verwendung von stöchiometrischem Kupfer(I)-cyanid in N,N-Dimethylformamid, N,N-Dimethylacetamid, N-Methylformanilid, N-Methylpyrrolidon, Hexamethylphosphorsäuretriamid und Dimethylsulfoxid als Lösungsmittel eine selektive Reaktion stattfindet und die Isolierung nach Entfernen der Kupfersalze mit wässrigem Ammoniak die cyanierten Ester der Formeln (II) und (III) in guten Ausbeuten bei entsprechender Reinheit liefert.

Die Durchführung des erfindungsgemäßen Verfahrensschritts erfolgt vorzugsweise innerhalb eines Temperaturbereichs von 100°C bis +200°C, besonders bevorzugt bei Temperaturen von 140°C bis + 180 °C.

Der erfindungsgemäße Verfahrensschritt (2) wird im Allgemeinen unter Normaldruck durchgeführt. Alternativ ist es jedoch auch möglich, im Vakuum zu arbeiten oder unter Druck.

Die Reaktionszeit ist nicht kritisch und kann, in Abhängigkeit von der Ansatzgröße und von der Temperatur, in einem Bereich zwischen unter einer und mehreren Stunden gewählt werden.

Bei der Durchführung des erfindungsgemäßen Verfahrensschritts setzt man für 1 Mol des Esters der Formel (IV) bzw. (V) 0,8 Mol bis 1,4 Mol, vorzugsweise 0,9 Mol bis 1,2 Mol, besonders bevorzugt 1,00 Mol Kupfer(I)-cyanid ein.

Geeignete Lösungsmittel sind beispielsweise aliphatische, alicyclische oder aromatische halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie 1,2-Dimethoxyethan, 1,2-Diethoxyethan oder Anisol; Nitrile, wie Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan; Pyridinderivate, wie Pyridin, 2-Methyl-5-ethylpyridin, 2-Picolin, 3-Picolin, besonders bevorzugt verwendet werden N,N-Dimethylformamid, N,N-Dimethylacetamid, N-Methylformanilid, N-Methylpyrrolidon, Pyridin, 2-Methyl-5-ethylpyridin, 2-Picolin, 3-Picolin.

Die cyanierten Produkte der Formeln (II) und (III) fallen zunächst als Kupferkomplexe an und können durch Waschen mit wässrigem Ammoniak, Ethylendiamin, Etylendiamintetraessigsäure (EDTA), wässrigem Eisen(III)-chlorid dekomplexiert und entweder aus dem wässrig anfallenden Feststoff oder extraktiv mit geeigneten Lösungsmitteln isoliert werden. Die erhaltenen Produkte der Formeln (II) und (III) können ohne weitere Aufreinigung im darauffolgenden Schritt (3), in welchem die Aminolyse stattfindet, eingesetzt werden.

### Schritt 3

### 2-Amino-5-cyano-N,3-dimethylbenzamid der Formel (I) kann folgendermaßen hergestellt werden:

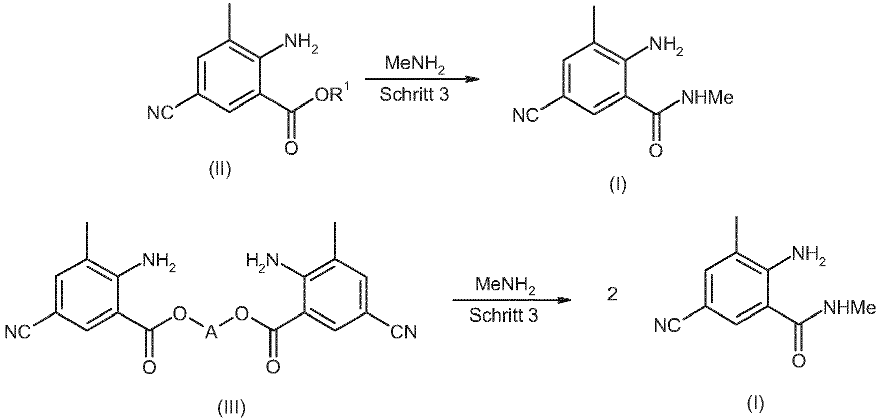

2-Amino-5-cyano-N,3-dimethylbenzamid der Formel (I) kann durch Umsetzung entsprechender 2-Amino-5-cyano-3-methylbenzoesäureester der Formel (II) bzw. -diester der Formel (III) mit Methylamin mit oder ohne katalytische Zusätzen einer Base in hoher Ausbeute und Reinheit erhalten werden. In der Literatur sind Aminolysen unter Zusatz von Natriummethanolat : R. L. Betts et al., J. Am. Chem. Soc. 1937, 59, 1568-1572; R. J. de Feoand et al., J. Org. Chem. 1963, 28, 2915-2917, Natriumamid, Natriumhydrid, Grignardreagentien und Butyllithium als Base beschrieben: Zit 4-8 in T. Högberg, J. Org. Chem. 1987, 52, 2033-2036. Auch Natriumcyanide als Base für Aminolysen sind in der Literatur beschrieben (T. Högberg, J. Org. Chem. 1987, 52, 2033-2036). Aminolysen von Cyanoanthranilsäurederivaten sind in WO 2006/062978 vorbeschrieben, allerdings ohne experimentelle Belege. Bei der Umsetzung von Cyanoanthranilsäureestern mit Methylamin in Methanol unter Zusatz von Natriummethanolat wurde ein vollständiger Umsatz bei hoher Ausbeute und Reinheit beobachtet. Die Reaktion kann auch ohne Zusatz an Natriummethanolat durchgeführt werden. Zudem erfolgt kein Angriff an der Nitrilfunktionalität unter Bildung unerwünschter Nebenprodukte.

Die Durchführung des erfindungsgemäßen Verfahrensschritts erfolgt vorzugsweise innerhalb eines Temperaturbereichs von 20°C bis +100°C, bevorzugt bei Temperaturen von 20°C bis 80°C und besonders bevorzugt bei Temperaturen von 20°C bis 60°C.

Der erfindungsgemäße Verfahrensschritt (3) wird im Allgemeinen unter Normaldruck durchgeführt. Alternativ ist es jedoch auch möglich unter Druck zu arbeiten.

Die Reaktionszeit ist nicht kritisch und kann, in Abhängigkeit von der Ansatzgröße und von der Temperatur, in einem Bereich zwischen unter einer und mehreren Stunden gewählt werden.

Die Reaktion wird bevorzugt batchweise durchgeführt. Kontinuierliche Reaktionsdurchführungen sind jedoch ebenfalls möglich.

Bei der Durchführung des erfindungsgemäßen Verfahrensschritts setzt man für 1 Mol des Esters der Formel (IV) bzw. (V) 2 Mol bis 20 Mol, vorzugsweise 5 Mol bis 15 Mol, besonders bevorzugt 10 Mol Methylamin ein.

Die Reaktion kann ohne Zusatz einer Base durchgeführt werden. Verwendet man eine Base, sind folgende geeignet: z. B. Natriumhydroxid, Kaliumcarbonat, Natriumcarbonat, Cäsiumcarbonat, Natriummethylat, Natriumamid, Grignardreagentien, Butyllithium, Triethylamin, Diisopropylethylamin oder Natriumhydrid. Besonders bevorzugt sind Alkoholatbasen (ROM, R = Alkyl, M = Na, K), Natriumhydroxid und Kaliumhydroxid.

Geeignet sind alle unter den Reaktionsbedingungen weitgehend inerten Lösungsmittel, beispielsweise aliphatische, alicyclische oder aromatische halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan, Alkohole wie Methanol, Ethanol, Isopropanol, sowie Lösungsmittelmischungen. Besonders bevorzugt verwendet man Acetonitril, Methanol, Ethanol, iso-Propanol und Butyronitril.

Das 2-Amino-5-cyano-N,3-dimethylbenzamid fällt gegen Ende der Reaktion als Niederschlag an und kann durch Filtration in Ausbeuten von ca 82 -90% bei Reinheiten von ca 93 - 95 Gewichts-% HPLC erhalten werden.

### Herstellbeispiele

Die folgenden Herstellbeispiele illustrieren die Erfindung, ohne sie zu beschränken.

### Beispiel 1

### 2-Amino-3-methyl-benzoesäuremethylester

¹H-NMR (600 MHz, (d⁶-DMSO): δ = 7.64 (dd, 1H), 7.19 (d, 1H), 6.50 (pq, 3H, Ar-H, NH2), 3.79 (s, 3H), 2.12 (s, 3H).

### 2-Amino-5-brom-3-methylbenzoesäuremethylester:

Zu einer Lösung von Methyl-2-amino-3-methyl-benzoat (142.1 g, 0.843 mol, Reinheit: 98 % quant.-NMR) in 240 mL H₂O wird bei 30°C Bromwasserstoff (48% in H₂O, 149.2 g, 0.885 mol) langsam zugetropft. Zur erhaltenen Suspension wird Wasserstoffperoxid (30 % in H₂O, 105.1 g, 0.927 mol) innerhalb 2 h zugetropft und die Temperatur unterhalb 70°C gehalten. Nach 1 Stunde Nachrührzeit wird NaHSO₃ (39 % in H₂O, 33.7 g, 0.126 mol) portionsweise zugegeben (Peroxidtest war negativ). Die erhaltene Suspension wird mit Na₂CO₃ (0.1 eq., 9.0 g, 0.084 mol) welches portionsweise zugegeben wird, auf pH =7-8 gestellt. Nach Filtration und Trocknen im Vakuumtrockenschrank wird Methyl-2-amino-5-brom-3-methylbenzoat als hellbrauner Feststoff isoliert. Ausbeute: 204.2 g, 97.7 % d. Th., Reinheit: 98.5 % quant.-NMR).
¹H-NMR (600 MHz, (d⁶-DMSO): δ = 7.70 (d, 1H), 7.36 (pt, 1H), 6.63 (br s, 2H), 3.80 (s, 3H), 2.12 (s, 3H)).

Analog zur oben beschriebenen Vorschrift **(Beispiel 1)** wurde Pentyl-2-amino-3-methylbenzoat (2.28 g, 10 mmol) mit Bromwasserstoff (2.21 g, 13.1 mmol, 48 % in Wasser) und Wasserstoffperoxid (1.72 g, 15.0 mmol, 30 % in Wasser) umgesetzt. Nach Aufarbeitung analog zu Beispiel 1 und zusätzlicher Säulenchromatographie Pentyl-2-amino-5-brom-3-methylbenzoat (2.7 g, 88.3% d. Th., >99 Flächen-% LC) als gelben Feststoff erhalten.
¹H-NMR (600 MHz, (CD₃CN): δ = 7.81 (d, 1H), 7.32 (pt, 1H), 6.10 (br s, 2H), 4.23 (t, 2 H), 2.12 (s, 3H), 1.74 (m, 2H), 1.45 (m, 4H), 0.93 (m, 3H).

Analog zur oben beschriebenen Vorschrift **(Beispiel 1)** wurde Hexyl-2-amino-3-methylbenzoat (2.50 g, 10 mmol) mit Bromwasserstoff (2.21 g, 13.1 mmol, 48 % in Wasser) und Wasserstoffperoxid (1.72 g, 15.0 mmol, 30 % in Wasser) umgesetzt. Nach Aufarbeitung analog zu Beispiel 1 und zusätzlicher Säulenchromatographie Hexyl-2-amino-5-brom-3-methylbenzoat (2.5 g, 78.8 % d. Th., >99 Flächen-% LC) als braunes Öl erhalten.
¹H-NMR (600 MHz, (CD₃CN): δ = 7.80 (d, 1H), 7.31 (d, 1H), 6.10 (br s, 2H), 4.23 (t, 2 H), 2.12 (s, 3H), 1.73 (m, 2H), 1.46 (m, 6H), 0.91 (m, 3H).

Analog zur oben beschriebenen Vorschrift **(Beispiel 1)** wurde 2-Ethylhexyl-2-amino-3-methylbenzoat (3.00 g, 10.6 mmol) mit Bromwasserstoff (2.33 g, 13.8 mmol, 48 % in Wasser) und Wasserstoffperoxid (1.81 g, 15.9 mmol, 30 % in Wasser) umgesetzt. Nach Aufarbeitung analog zu Beispiel 1 und zusätzlicher Säulenchromatographie 2-Ethylhexyl-2-amino-5-brom-3-methylbenzoat (2.7 g, 74.0 % d. Th., >99 Flächen-% LC) als hellbraunes Öl erhalten.
¹H-NMR (600 MHz, (CD₃CN): δ = 7.79 (d, 1H), 7.32 (d, 1H), 6.11 (br s, 2H), 4.17 (d, 2H), 2.13 (s, 3H), 1.70 (m, 7H), 0.94 (m, 7H).

Analog zur oben beschriebenen Vorschrift **(Beispiel 1)** wurde 2-Amino-3-methylbenzoesäureethyl-ester (16.29 g, 90.0 mmol) mit Bromwasserstoff (15.93 g, 94.5 mmol, 48 % in Wasser) und Wasserstoffperoxid (11.25 g, 99.0 mmol, 30 % in Wasser) umgesetzt und nach Aufarbeitung analog zu Beispiel 1 Ethyl-2-amino-5-brom-3-methylbenzoat (20.7 g, 89.1 % d. Th., >99 Flächen-% LC) als braunes Öl erhalten.
¹H-NMR (600 MHz, (CD₃CN): δ = 7.81 (d, 1H), 7.32 (pq, 1H), 6.10 (br s, 2H), 4.28 (q, 2H), 1.34 (t, 3H).

Analog zur oben beschriebenen Vorschrift **(Beispiel 1)** wurde Ethan-1,2-diylbis(2-Amino-3-methylbenzoat (2.0 g, 5.89 mmol) mit Bromwasserstoff (2.09 g, 12.3 mmol, 48 % in Wasser) und Wasserstoffperoxid (1.47 g, 12.9 mmol, 30 % in Wasser) umgesetzt und nach Aufarbeitung analog zu Beispiel 1 Ethan-1,2-diylbis(2-Amino-5-brom-3-methylbenzoat) (2.4 g, 80.0 % d. Th., 95.5 Flächen-% LC) als hellgelber Feststoff erhalten.
¹H-NMR (600 MHz, (d⁶-DMSO): δ = 7.71 (d, 1H), 7.36 (d, 1H), 6.63 (br s, 2H), 4.56 (s, 2H), 2.11 (s, 3H).

Analog zur oben beschriebenen Vorschrift **(Beispiel 1)** wurde Hexan-1,6-diylbis(2-amino-3-methylbenzoat) (4.0 g, 10.4 mmol) mit Bromwasserstoff (3.57 g, 21.1 mmol, 48 % in Wasser) und Wasserstoffperoxid (2.52 g, 22.1 mmol, 30 % in Wasser) umgesetzt und nach Aufarbeitung analog zu Beispiel 1 Hexan-1,6-diylbis(2-amino-5-brom-3-methylbenzoat) (5.36 g, 90.9 % d. Th., 92.6 Flächen-% LC) als brauen Feststoff erhalten.
¹H-NMR (600 MHz, (d⁶-DMSO): δ = 7.69 (d, 1H), 7.35 (d, 1H), 6.63 (br s, 2H), 4.22 (t, 2H), 2.11 (s, 3H), 1.73 (m, 2H), 1.45 (m, 2H).

Analog zur oben beschriebenen Vorschrift **(Beispiel 1)** wurde 2-Methoxyethyl-2-amino-3-methylbenzoat (7.00 g, 33.4 mmol) mit Bromwasserstoff (5.92 g, 35.1 mmol, 48 % in Wasser) und Wasserstoffperoxid (4.17 g, 36.7 mmol, 30 % in Wasser) umgesetzt und nach Aufarbeitung analog zu Beispiel 1 2-Methoxyethyl-2-amino-5-brom-3-methylbenzoat (8.50 g, 80.7 % d. Th., 91.5 Flächen-% LC) als brauen Feststoff erhalten.
¹H-NMR (600 MHz, (d⁶-DMSO): δ = 7.70 (d, 1H), 7.37 (d, 1H), 6.63 (br s, 2H), 4.34 (t, 2H), 3.65 (t, 2H), 3.31 (s, 3H), 2.10 (s, 3H)).

Eine Lösung von Methyl-2-amino-5-brom-3-methylbenzoat (5 g, 20.4 mmol) in Benzylalkohol (4.43 g, 40.9 mmol) und Natriummethanolat (0.37 g, 2.04 mmol, 30 %ig in Methanol) wurde für 5 Stunden in Xylol (10 ml) unter Refluxieren erhitzt. Dann wurde Wasser hinzugefügt, die Phasen getrennt und die vereinigten organischen Lösungsmittel wurden im Vakuum entfernt. Der Rückstand wurde säulenchromatographisch aufgereinigt. Man erhielt Benzyl-2-amino-5-brom-3-methylbenzoat (3.40 g, 43.3 % d. Th., 83.6 Flächen-% GC) als hellbraunen Feststoff.
¹H-NMR (600 MHz, (CDCl₃): δ = 7.92 (s, 1H), 7.44-7.28 (m, 6H), 5.86 (s, 2H), 5.33 (s, 2H), 2.14 (s, 3H).

### Beispiel 2

Methyl-2-amino-5-brom-3-methylbenzoat (100 g, 0.393 mol, 96 % quant.-NMR), Kupfer(I)-cyanid (36.3 g, 0.401 mol) und N-Methyl-2-pyrrolidinon (NMP) (206 g, 200 mL, 2.084 mol) werden unter Rühren für 4 Stunden auf 170°C erhitzt. Der Reaktionsansatz wird auf 120°C abgekühlt, 350 mL H₂O (90°C) innerhalb von 30 Minuten zugetropft und die erhaltene Suspension wird filtriert. Der erhaltene Feststoff wird mit zweimal mit Ammoniak (200 g, 12 % in H₂O) und 2mal mit 100 ml Wasser gewaschen. Nach Trocknen im Vakuumtrockenschrank bei 50°C wird Methyl-2-amino-5-cyano-3-methylbenzoat als grauen Feststoff erhalten. Ausbeute: 69.0 g, 88.2 % d. Th., Reinheit: 95.6 % quant.-NMR, 1500 ppm Cu).
¹H-NMR (600 MHz, (MeOD): δ = 8.04 (d, 1H), 7.40 (m, 1H), 4.85 (s, 3H), 2.18 (s, 3H).

Analog zur oben beschriebenen Vorschrift **(Beispiel 2)** wurde Pentyl-2-amino-5-brom-3-methylbenzoat (2.3 g, 7.59 mmol) mit Kupfercyanid (0.69 g, 7.74 mmol) in N,N-Dimethylacetamid 6 Stunden bei 170°C umgesetzt. Nach Aufarbeitung analog zu Beispiel 2 und zusätzlicher Extraktion mit Ethylacetat und Waschen mit wässriger 5% Ethylendiamin-Lösung wurde Pentyl-2-amino-5-cyan-3-methylbenzoat (1.78 g, 89.2 % d. Th., 93.6 Flächen-% LC) als braunes Öl erhalten.
¹H-NMR (600 MHz, (CD₃CN): δ = 8.08 (d, 1H), 7.43 (s, 1H), 6.67 (br s, 2H), 4.24 (t, 2H), 2.15 (s, 3H), 1.75 (m, 2H), 1.40 (m, 4H), 1.05 (m, 3H).

Analog zur oben beschriebenen Vorschrift **(Beispiel 2)** wurde Hexyl-2-amino-5-brom-3-methylbenzoat (2.2 g, 6.79 mmol) mit Kupfercyanid (0.62 g, 6.93 mmol) in N,N-Dimethylacetamid 6 Stunden bei 170°C umgesetzt. Nach Aufarbeitung analog zu Beispiel 2 und zusätzlicher Extraktion mit Etylacetat und Waschen mit wässriger 5% Etylendiamin-Lösung Hexyl-2-amino-5-cyan-3-methylbenzoat (1.66 g, 85.5 % d. Th., 91.1 Flächen-% LC) als braunes Öl erhalten.
¹H-NMR (600 MHz, (CD₃CN): δ = 8.08 (d, 1H), 7.43 (s, 1H), 6.67 (br s, 2H), 4.24 (t, 2H), 2.14 (s, 3H), 1.74 (m, 2H), 1.39 (m, 6H), 0.92 (m, 3H).

Analog zur oben beschriebenen Vorschrift **(Beispiel 2)** wurde 2-Ethylhexyl-2-amino-5-brom-3-methylbenzoat (2.00 g, 5.84 mmol) mit Kupfercyanid (0.53 g, 5.96 mmol) in N,N-Dimethylacetamid 8 Stunden bei 160°C umgesetzt. Nach Aufarbeitung analog zu Beispiel 2 und zusätzlichem Waschen mit wässriger 5% Etylendiamin-Lösung wurde 2-Ethylhexyl 2-amino-5-cyan-3-methylbenzoat (1.45 g, 76.7 % d. Th., 89.1 Flächen-% LC) als braunes Öl erhalten.
¹H-NMR (400 MHz, CD₃CN): δ = 8.06 (d, 1H), 7.43 (s, 1H), 6.70 (br. s, 2H), 4.19 (d, 2H), 2.15 (s, 3H), 1.71 (m, 1H), 1.39 (m, 8 H), 0.90 (m, 6H).

Analog zur oben beschriebenen Vorschrift **(Beispiel 2)** wurde Ethyl-2-amino-5-brom-3-methylbenzoat (2.00 g, 7.65 mmol) mit Kupfercyanid (0.70 g, 7.80 mmol) in N,N-Dimethylacetamid 8 Stunden bei 160°C umgesetzt. Nach Aufarbeitung analog zu Beispiel 2 wurde Ethyl-2-amino-5-cyan-3-methylbenzoat (1.30 g, 80.2 % d. Th., 96.3 Flächen-% LC) als braunen Feststoff erhalten.
¹H-NMR (400 MHz, CD₃CN): δ = 8.09 (d, 1H), 7.43 (s, 1H), 6.68 (br. s, 2H), 4.30 (q, 2H), 2.13 (s, 3H), 1.35 (t, 3H).

Analog zur oben beschriebenen Vorschrift **(Beispiel 2)** wurde Ethan-1,2-diylbis(2-amino-5-brom-3-methylbenzoat) (2.00 g, 3.94 mmol) mit Kupfercyanid (0.72 g, 8.07 mmol) in N-Methylpyrrolidon 4 Stunden bei 170°C umgesetzt. Nach Aufarbeitung analog zu Beispiel 2 und zusätzlichem Waschen mit wässriger 5% Etylendiamin-Lösung wurde Ethan-1,2-diylbis(2-amino-5-cyan-3-methylbenzoat) (1.54 g, 81.9 % d. Th., 79.2 Flächen-% LC) als braunen Feststoff erhalten.
¹H-NMR (600 MHz, d⁶-DMSO): δ = 8.00 (d, 1H), 7.54 (s, 1H), 7.27 (br s, 2H), 4.60 (s, 2H), 2.12 (s, 3H).

Analog zur oben beschriebenen Vorschrift **(Beispiel 2)** wurde Hexan-1,6-diylbis(2-amino-5-brom-3-methylbenzoat) (4.00 g, 6.86 mmol) mit Kupfercyanid (1.29 g, 14.06 mmol) in N-Methylpyrrolidon 4 Stunden bei 170°C umgesetzt. Nach Aufarbeitung analog zu Beispiel 2 und zusätzlichem Waschen mit wässriger 5% Etylendiamin-Lösung wurde Hexan-1,2-diylbis(2-amino-5-cyan-3-methylbenzoat) (3.20 g, 82.7 % d. Th., 77.0 Flächen-% LC) als braunen Feststoff erhalten.
¹H-NMR (600 MHz, (d⁶-DMSO): δ = 7.98 (d, 1H), 7.53 (s, 1H), 7.28 (br s, 2H), 4.25 (t, 2H), 2.14 (s, 3H), 1.74 (m, 2H), 1.46 (m, 2H).

Analog zur oben beschriebenen Vorschrift **(Beispiel 2)** wurde 2-Methoxyethyl-2-amino-5-brom-3-methylbenzoat (5.00 g, 15.88 mmol) mit Kupfercyanid (1.49 g, 16.67 mmol) in N-Methylpyrrolidon 6 Stunden bei 170°C umgesetzt. Nach Aufarbeitung analog zu Beispiel 2 und zusätzlicher Extraktion mit Etylacetat und Waschen mit wässriger 5% Etylendiamin-Lösung wurde 2-Methoxyethyl-2-amino-5-cyan-3-methylbenzoat (0.45 g, 11.6 % d. Th., 95.6 Flächen-% LC) als braunen Feststoff erhalten.
¹H-NMR (600 MHz, (CD₃CN): δ = 8.08 (d, 1H), 7.45 (s, 1H), 6.67 (br s, 2H), 4.38 (t, 2H), 3.68 (t, 2H), 3.36 (s, 3H), 2.15 (s, 3H).

Analog zur oben beschriebenen Vorschrift **(Beispiel 2)** wurde Benzyl-2-amino-5-brom-3-methylbenzoat (3.00 g, 7.38 mmol) mit Kupfercyanid (0.772 g, 7.98 mmol) in N-Methylpyrrolidon 6 Stunden bei 170°C umgesetzt. Nach Aufarbeitung analog zu Beispiel 2, zusätzlicher Extraktion mit Etylacetat, Waschen mit wässriger 5% Etylendiamin-Lösung und chromatographischer Aufreinigung wurde Benzyl-2-amino-5-cyan-3-methylbenzoat (1.00 g, 47.9 % d. Th., 97.0 Flächen-% LC) als hellbrauner Feststoff erhalten.
¹H-NMR (600 MHz, (CDCl₃): δ = 8.16 (s, 1H), 7.44-7.35 (m, 6H), 6.40 (s, 2H), 5.33 (s, 2H), 2.17 (s, 3H).

### Beispiel 3

### 2-Amino-5-cyano-N,3-dimethylbenzamid

In eine Lösung von 2-Amino-5-cyano-3-methylbenzoesäureethylester (50 g, 0.251 mol) in Methanol (175 mL) wird Methylamin (117 g, 3.77 mol) eingeleitet, Natriummethanolat (1.13 g, 30 %ig in Methanol, 6.28 mmol) zugegeben und das Reaktionsgemisch über Nacht bei Raumtemperatur gerührt. Danach werden 100 ml 22 %ige NaOH zugefügt und der Ansatz auf 10°C abgekühlt. Die erhaltene Suspension wird filtriert und der erhaltene Feststoff einmal mit Methanol : Wasser = 1: 1 und anschließend mit 50 mL Wasser gewaschen. Der Feststoff im Vakuumtrockenschrank bei 50°C getrocknet. 2-Amino-5-cyano-N,3-dimethylbenzamid wird als schwach beiger Feststoff isoliert. Ausbeute: 80.3 % d. Th. , Reinheit: 94.9 % quant.-LC, 73 ppm Cu).
¹H-NMR (600 MHz, (CD₃CN): δ = 7.65 (d, 1H), 7.38 (s, 1H), 6.91 (br s, 1H), 6.52 (br s, 2 H), 2.82 (d, 3H), 2.12 (s, 3H).

Analog zur oben beschriebenen Vorschrift **(Beispiel 3)** wurde Pentyl-2-amino-5-cyan-3-methylbenzoat (1.50 g) mit Methylamin (10.8 g, 40 %ig in Methanol) und Natriummethanolat (441 mg, 0.12 mmol, 30 %ig in Methanol) für 18 Stunden bei Raumtemperatur gerührt. Der gesamte Reaktionsansatz wurde im Vakuum destilliert und mit Diisopropylether verrührt. 2-Amino-5-cyano-N,3-dimethylbenzamid (0.79 g, 70.6 % d. Th., 96.4 Flächen-% LC) wurde als braunen Feststoff erhalten.

Analog zur oben beschriebenen Vorschrift **(Beispiel 3)** wurde Hexyl-2-amino-5-cyan-3-methylbenzoat (1.40 g, 4.89 mmol) mit Methylamin (10.8 g, 139 mmol, 40 %ig in Methanol) und Natriummethanolat (441 mg, 0.12 mmol, 30 %ig in Methanol) für 18 Stunden bei Raumtemperatur gerührt. Der gesamte Reaktionsansatz wurde im Vakuum destilliert und mit Diisopropylether verrührt. 2-Amino-5-cyano-N,3-dimethylbenzamid (0.75 g, 75.8 % d. Th., 93.6 Flächen-% LC) wurde als braunen Feststoff erhalten.

Analog zur oben beschriebenen Vorschrift **(Beispiel 3)** wurde 2-Ethylhexyl-2-amino-5-cyan-3-methylbenzoat (1.00 g, 3.09 mmol) mit Methylamin (3.60 g, 46.3 mmol, 40 %ig in Methanol) und einem Tropfen Natriummethanolat (30 %ig in Methanol) für 18 Stunden bei Raumtemperatur gerührt. Der gesamte Reaktionsansatz wurde im Vakuum destilliert und 2-Amino-5-cyano-N,3-dimethyl-benzamid (730 mg, 91.4 % d. Th., 73.1 Flächen-% LC) als braunen Feststoff erhalten.

Analog zur oben beschriebenen Vorschrift **(Beispiel 3)** wurde Ethyl-2-amino-5-cyan-3-methylbenzoat (0.50 g, 2.40 mmol) mit Methylamin (3.73 g, 48 mmol, 40 %ig in Methanol) und Natriummethanolat (22 mg, 30 %ig in Methanol) für 18 Stunden bei Raumtemperatur gerührt. Der gesamte Reaktionsansatz wurde im Vakuum destilliert und 2-Amino-5-cyano-N,3-dimethylbenzamid (0.44 g, 95.9 % d. Th., 99.0 Flächen-% LC) als hellbraunen Feststoff erhalten.

Analog zur oben beschriebenen Vorschrift **(Beispiel 3)** wurde Ethan-1,2-diylbis(2-amino-5-cyan-3-methylbenzoat) (20 mg, 0.04 mmol) mit Methylamin (5.40 g, 69.5 mmol, 40 %ig in Methanol) und einem Tropfen Natriummethanolat (30 %ig in Methanol) für 18 Stunden bei Raumtemperatur gerührt. Der gesamte Reaktionsansatz wurde im Vakuum destilliert und nach chromatographischer Aufreinigung 2-Amino-5-cyano-N,3-dimethylbenzamid (8 mg, 47.2 % d. Th., 93.1 Flächen-% LC) als braunen Feststoff erhalten.

Analog zur oben beschriebenen Vorschrift **(Beispiel 3)** wurde Hexan-1,6-diylbis(2-amino-5-brom-3-methylbenzoat) (220 mg, 0.4 mmol) mit Methylamin (2 g, 25.7 mmol, 40 %ig in Methanol) und einem Tropfen Natriummethanolat (30 %ig in Methanol) für 18 Stunden bei Raumtemperatur gerührt. Der gesamte Reaktionsansatz wurde im Vakuum destilliert und nach chromatographischer Aufreinigung 2-Amino-5-cyano-N,3-dimethylbenzamid (61 mg, 50.5 % d. Th., 94.6 Flächen-% LC) als braunen Feststoff erhalten.

Analog zur oben beschriebenen Vorschrift **(Beispiel 3)** wurde 2-Methoxyethyl-2-amino-5-cyan-3-methylbenzoat (200 mg, 0.81 mmol) mit Methylamin (5.40 g, 69.5 mmol, 40 %ig in Methanol) und einem Tropfen Natriummethanolat (30 %ig in Methanol) für 18 Stunden bei Raumtemperatur gerührt. Der gesamte Reaktionsansatz wurde im Vakuum destilliert und nach chromatographischer Aufreinigung 2-Amino-5-cyano-N,3-dimethylbenzamid (150 mg, 95.0 % d. Th., 97.8 Flächen-% LC) als hellbraunen Feststoff erhalten.

Analog zur oben beschriebenen Vorschrift **(Beispiel 3)** wurde 2-Benzyl-2-amino-5-cyan-3-methylbenzoat (1.00 g, 3.75 mmol) mit Methylamin (4.78 g, 61.6 mmol, 40 %ig in Methanol) und einem Tropfen Natriummethanolat (30 %ig in Methanol) für 18 Stunden bei Raumtemperatur gerührt. Der gesamte Reaktionsansatz wurde im Vakuum destilliert und nach chromatographischer Aufreinigung 2-Amino-5-cyano-N,3-dimethylbenzamid (616 mg, 85.4 % d. Th., 98.4 Flächen-% LC) als farblosen Feststoff erhalten.

## Patentansprüche

1. Verfahren zur Herstellung von 2-Amino-5-cyano-N,3-dimethylbenzamid der Formel (I) 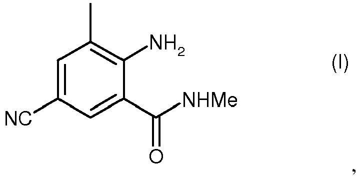 **dadurch gekennzeichnet, dass**
(A1) Verbindungen der Formel (VI) 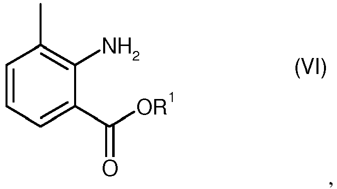 in welcher
R¹ für Alkyl, Cycloalkyl, Alkoxyalkyl, Arylalkyl, Thioalkyl, Alkylthioalkyl, Alkylsulfonylalkyl, Cyanoalkyl, Nitroalkyl, Haloalkyl oder Aryl steht,
mit einer Mischung aus HBr/H₂O₂ in Wasser umgesetzt werden zu Verbindungen der Formel (IV) 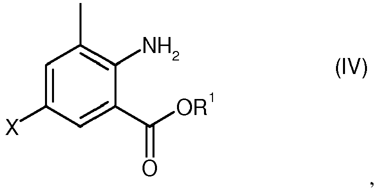 in welcher
X für Br steht und R¹ die oben angegebenen Bedeutungen hat,
(A2) diese Verbindungen der Formel (IV) mit Kupfer(I)-cyanid in N,N-Dimethylformamid, N,N-Dimethylacetamid, N-Methylformanilid, N-Methylpyrrolidon, Hexamethylphosphorsäuretriamid oder Dimethylsulfoxid zu 2-Amino-5-cyano-3-methylbenzoesäureester der Formel (II) 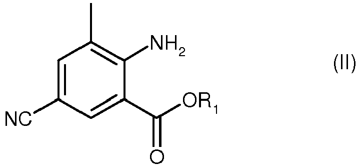 umgesetzt werden;
(A3) diese 2-Amino-5-cyano-3-methylbenzoesäureester der Formel (II) mit MeNH₂ unter Verwendung von mindestens einer Base umgesetzt werden zu 2-Amino-5-cyano-N,3-dimethylbenzamid der Formel (I);
oder
(B1) Verbindungen der Formel (VII), 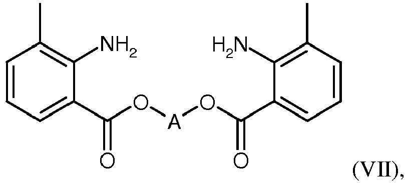 in welcher
A für Alkylen, Alkoxyalkylen steht,
mit einer Mischung aus HBr/H₂O₂ in Wasser umgesetzt werden zu Verbindungen der Formel (V) 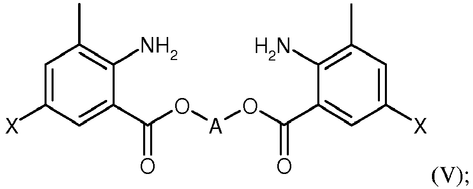
(B2) diese Verbindungen der Formel (V) mit Kupfer(I)-cyanid in N,N-Dimethylformamid, N,N-Dimethylacetamid, N-Methylformanilid, N-Methylpyrrolidon, Hexamethylphosphorsäuretriamid oder Dimethylsulfoxid umgesetzt werden zu 2-Amino-5-cyano-3-methylbenzoesäurediester der Formel (III) 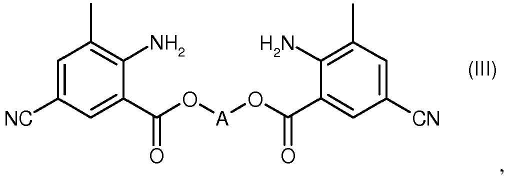 in welcher A für Alkylen, Alkoxyalkylen steht,
(B3) diese Verbindungen der Formel (III) mit MeNH₂ unter Verwendung von mindestens einer Base umgesetzt werden zu 2-Amino-5-cyano-N,3-dimethylbenzamid der Formel (I).

2. Verfahren gemäß Anspruch 1, **dadurch gekennzeichnet, dass** die Verbindung der Formel (I) durch Umsetzung von 2-Amino-5-cyano-3-methylbenzoesäureestern der Formel (II) erhalten wird.

3. Verfahren gemäß Anspruch 1, **dadurch gekennzeichnet, dass** die Verbindung der Formel (I) durch Umsetzung von 2-Amino-5-cyano-3-methylbenzoesäurediestern der Formel (III) erhalten wird.

4. Verfahren gemäß einem der Ansprüche1, 2 oder 3, **dadurch gekennzeichnet, dass** die Base ausgewählt ist aus der Gruppe bestehend aus Natriumamid, Natriumhydrid, Natriumhydroxid, Natriumcyanid, Kaliumcyanid, Kaliumcarbonat, Natriumcarbonat, Cäsiumcarbonat, Natriummethylat und Triethylamin.

5. Verfahren gemäß einem der Ansprüche 6 oder 7, **dadurch gekennzeichnet, dass** auf 1 Mol der Verbindung der Formel (IV) bzw. (V) 0,8 Mol bis 1,4 Mol Kupfer(I)-cyanid eingesetzt werden.

6. Verbindungen der allgemeinen Formel (II), 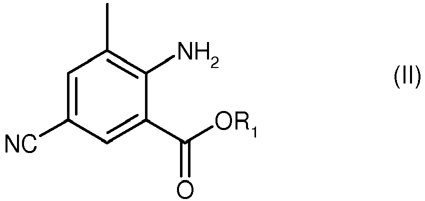 in welcher R¹ für Pentyl, Hexyl, oder 2-Ethylhexyl steht.

7. Verbindungen der allgemeinen Formel (III), 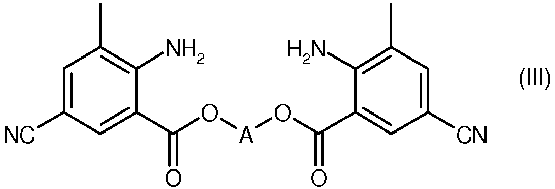 in welcher A für Ethylen oder Hexylen steht.

## Claims

1. Process for preparing 2-amino-5-cyano-N,3-dimethylbenzamide of formula (I) 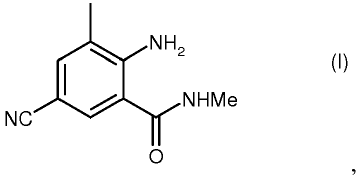 **characterized in that**
(A1) compounds of formula (VI) 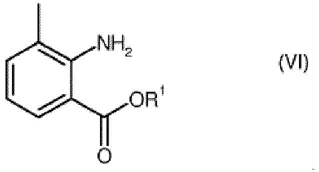 where
R¹ represents alkyl, cycloalkyl, alkoxyalkyl, arylalkyl, thioalkyl, alkylthioalkyl, alkylsulfonylalkyl, cyanoalkyl, nitroalkyl, haloalkyl or aryl,
are reacted with a mixture of HBr/H₂O₂ in water to form compounds of formula (IV) 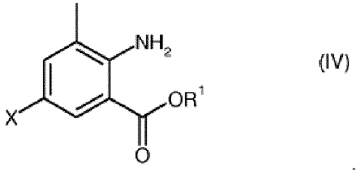 where
X represents Br and R¹ is as defined above,
(A2) these compounds of formula (IV) are reacted with copper(I) cyanide in N,N-dimethylformamide, N,N-dimethylacetamide, N-methylformanilide, N-methylpyrrolidone, hexamethylphosphoramide or dimethyl sulfoxide to form 2-amino-5-cyano-3-methylbenzoic esters of formula (II) 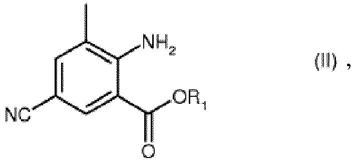
(A3) these 2-amino-5-cyano-3-methylbenzoic esters of formula (II) are reacted with MeNH₂ by use of at least one base to form 2-amino-5-cyano-N,3-dimethyl-benzamide of formula (I);
or
(B1) compounds of formula (VII), 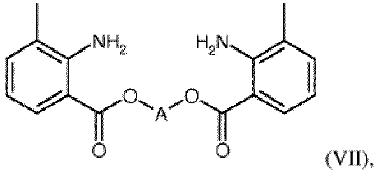 where
A represents alkylene or alkoxyalkylene,
are reacted with a mixture of HBr/H₂O₂ in water to form compounds of formula (V) 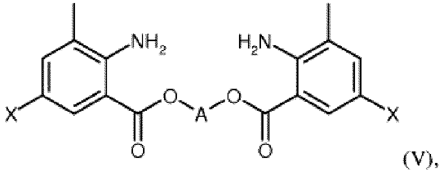
(B2) these compounds of formula (V) are reacted with copper(I) cyanide in N,N-dimethylformamide, N,N-dimethylacetamide, N-methylformanilide, N-methylpyrrolidone, hexamethylphosphoramide or dimethyl sulfoxide to form 2-amino-5-cyano-3-methylbenzoic diesters of formula (III) 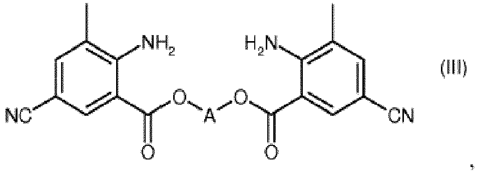 where A represents alkylene or alkoxyalkylene,
(B3) these compounds of formula (III) are reacted with MeNH₂ by use of at least one base to form 2-amino-5-cyano-N,3-dimethylbenzamide of formula (I).

2. Process according to Claim 1, **characterized in that** the compound of formula (I) is obtained by reacting 2-amino-5-cyano-3-methylbenzoic esters of formula (II).

3. Process according to Claim 1, **characterized in that** the compound of formula (I) is obtained by reacting 2-amino-5-cyano-3-methylbenzoic diesters of formula (III).

4. Process according to any one of Claims 1, 2 and 3, **characterized in that** the base is selected from the group consisting of sodium amide, sodium hydride, sodium hydroxide, sodium cyanide, potassium cyanide, potassium carbonate, sodium carbonate, caesium carbonate, sodium methoxide and triethylamine.

5. Process according to either Claim 6 or 7, **characterized in that** from 0.8 mol to 1.4 mol of copper(I) cyanide are used per 1 mol of compound of formula (IV) or (V).

6. Compounds of general formula (II), 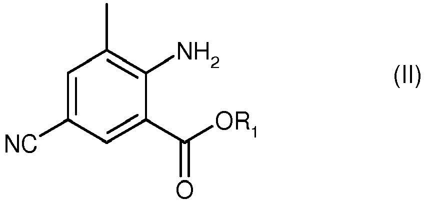 where R¹ represents pentyl, hexyl or 2-ethylhexyl.

7. Compounds of general formula (III), 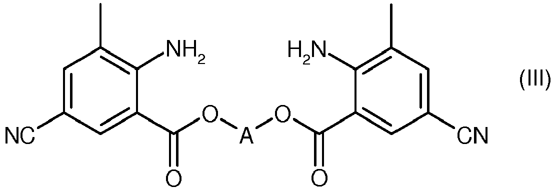 where A represents ethylene or hexylene.

## Revendications

1. Procédé de fabrication de 2-amino-5-cyano-N,3-diméthylbenzamide de formule (I) 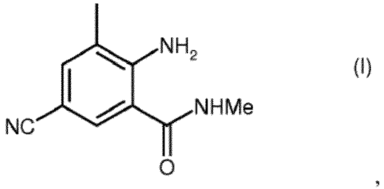 **caractérisé en ce que**
(A1) des composés de formule (VI) 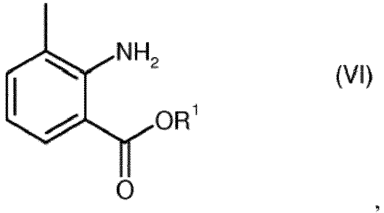 dans laquelle
R¹ représente alkyle, cycloalkyle, alcoxyalkyle, arylalkyle, thioalkyle, alkylthioalkyle, alkylsulfonylalkyle, cyanoalkyle, nitroalkyle, haloalkyle ou aryle,
sont mis en réaction avec un mélange d'HBr/H₂O₂ dans de l'eau pour former des composés de formule (IV) 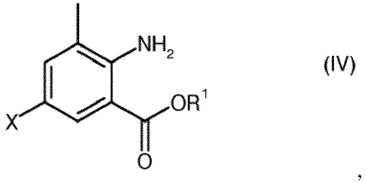 dans laquelle
X représente Br et R¹ a les significations indiquées précédemment,
(A2) ces composés de formule (IV) sont mis en réaction avec du cyanure de cuivre (I) dans du N,N-diméthylformamide, du N,N-diméthylacétamide, du N-méthylformanilide, de la N-méthylpyrrolidone, du triamide de l'acide hexaméthylphosphorique ou du diméthylsulfoxyde pour former des esters de l'acide 2-amino-5-cyano-3-méthylbenzoïque de formule (II) 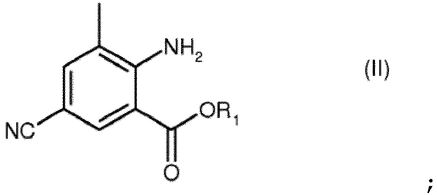
(A3) ces esters de l'acide 2-amino-5-cyano-3-méthylbenzoïque de formule (II) sont mis en réaction avec MeNH₂ en utilisant au moins une base pour former du 2-amino-5-cyano-N,3-diméthylbenzamide de formule (I) ; ou
(B1) des composés de formule (VII) 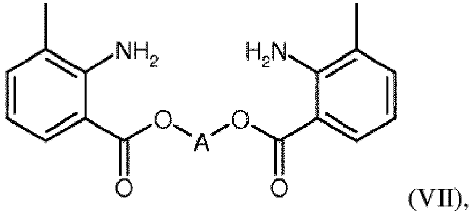 dans laquelle
A représente alkylène, alcoxyalkylène,
sont mis en réaction avec un mélange d'HBr/H₂O₂ dans de l'eau pour former des composés de formule (V) 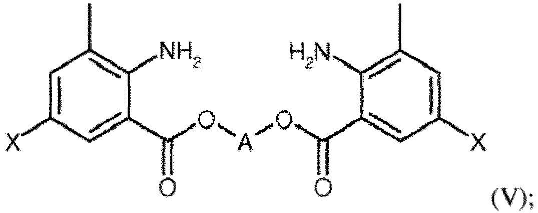
(B2) ces composés de formule (V) sont mis en réaction avec du cyanure de cuivre (I) dans du N,N-diméthylformamide, du N,N-diméthylacétamide, du N-méthylformanilide, de la N-méthylpyrrolidone, du triamide de l'acide hexaméthylphosphorique ou du diméthylsulfoxyde pour former des diesters de l'acide 2-amino-5-cyano-3-méthylbenzoïque de formule (III) 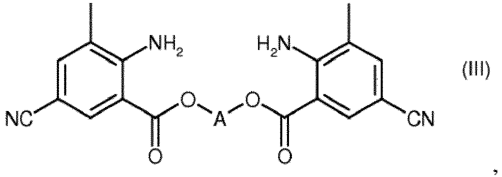 dans laquelle A représente alkylène, alcoxyalkylène,
(B3) ces composés de formule (III) sont mis en réaction avec MeNH₂ en utilisant au moins une base pour former du 2-amino-5-cyano-N,3-diméthylbenzamide de formule (I).

2. Procédé selon la revendication 1, **caractérisé en ce que** le composé de formule (I) est obtenu par mise en réaction d'esters de l'acide 2-amino-5-cyano-3-méthylbenzoïque de formule (II).

3. Procédé selon la revendication 1, **caractérisé en ce que** le composé de formule (I) est obtenu par mise en réaction de diesters de l'acide 2-amino-5-cyano-3-méthylbenzoïque de formule (III).

4. Procédé selon l'une quelconque des revendications 1, 2 ou 3, **caractérisé en ce que** la base est choisie dans le groupe constitué par l'amide de sodium, l'hydrure de sodium, l'hydroxyde de sodium, le cyanure de sodium, le cyanure de potassium, le carbonate de potassium, le carbonate de sodium, le carbonate de césium, le méthylate de sodium et la triéthylamine.

5. Procédé selon l'une quelconque des revendications 6 ou 7, **caractérisé en ce que**, pour 1 mole du composé de formule (IV) ou (V), 0,8 mole à 1,4 mole de cyanure de cuivre (I) est utilisé.

6. Composés de formule générale (II) 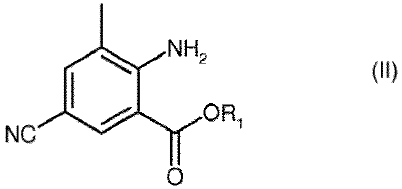 dans laquelle R¹ représente pentyle, hexyle ou 2-éthylhexyle.

7. Composés de formule générale (III) 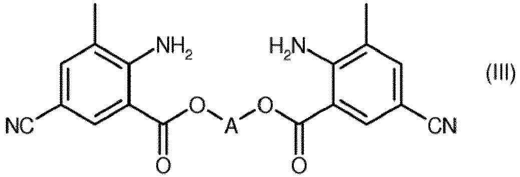 dans laquelle A représente éthylène ou hexylène.
